# DEMANDE DE BREVET EUROPEEN

(11) **EP 4 556 505 A2**
(43) Date de publication de la demande: **21.05.2025**
(21) Numéro de dépôt: 25161215.6
(22) Date de dépôt: 27.01.2020
(51) Int. Cl.: C08G 63/02

(54) **PROCÉDÉ DE PRODUCTION D'UN POLYESTER TÉRÉPHTALATE À PARTIR D'UN MÉLANGE MONOMÉRIQUE COMPRENANT UN DIESTER**

(30) Priorité: 01.02.2019 FR 1901023
(62) Demande divisionnaire de: 20701204.8
(71) Demandeur: IFP Energies nouvelles, 92500 Rueil-Malmaison (FR)
(72) Inventeur: THINON, Olivier, 92852 RUEIL-MALMAISON CEDEX (FR); GAUTHIER, Thierry, 92852 RUEIL-MALMAISON CEDEX (FR)
(74) Mandataire: IFP Energies nouvelles

(57) **Abrégé**

L'invention a pour objet un procédé de production d'un polyester téréphtalate, comprenant une étape a) de préparation d'une charge d'estérification comprenant au moins une section de mélange alimentée en au moins une charge acide téréphtalique et une charge monomère diester, de sorte que le rapport du nombre total de moles de motifs diol introduits dans ladite section de mélange, par rapport au nombre total de moles de motifs téréphtalate introduits dans ladite section de mélange, est compris entre 1,0 et 2,0, ladite section de mélange étant opérée à une température comprise entre 25 et 250°C et à une pression supérieure ou égale à 0,1 MPa, une étape b) d'estérification pour produire au moins un effluent réactionnel et un effluent aqueux, une étape c) de polycondensation pour obtenir au moins ledit polyester téréphtalate et un effluent comprenant au moins un monomère diol et une étape d) de traitement des diols pour obtenir un flux diol purifié.

## Description

### Domaine technique

L'invention concerne un procédé de production d'un polyester, en particulier d'un polyester téréphtalate, comme le polyéthylène téréphtalate (PET), à partir d'un mélange comprenant au moins l'acide téréphtalique, et un diester de l'acide téréphtalique et d'un diol. Ledit diester est de préférence un diester issu d'un procédé de recyclage de matière polyester, c'est-à-dire un diester obtenu à l'issu d'un procédé de dépolymérisation d'une matière polyester à recycler.

### Technique antérieure

Le recyclage chimique de polyester, en particulier du polyéthylène téréphtalate (PET), a fait l'objet de nombreux travaux visant à décomposer le polyester récupéré sous forme de déchets en monomères qui pourront de nouveau être utilisés comme charge d'un procédé de polymérisation.

De nombreux polyesters sont issus de circuits de collecte et de tri de matières. En particulier, le polyester, en particulier le PET, peut provenir de la collecte de bouteilles, barquettes, films, résines et/ou fibres composées de polyester (comme par exemple des fibres textiles, des fibres de pneus). Le polyester issu de filières de collecte et de tri est appelé polyester à recycler.

Le PET à recycler peut être classé en quatre grandes catégories :
- le PET clair, constitué majoritairement de PET transparent incolore (en général au moins 60% poids) et de PET transparent coloré azuré, qui ne contient pas de pigments et peut être engagé dans des procédés de recyclage mécanique,
- le PET foncé, ou coloré (vert, rouge,..), qui peut contenir généralement jusqu'à 0,1% poids de colorants ou pigments mais reste transparent, ou translucide ;
- le PET opaque, qui contient une quantité significative de pigments à des teneurs variant typiquement entre 0,25 et 5,0% poids pour opacifier le polymère. Le PET opaque est utilisé de manière grandissante par exemple pour la fabrication de contenants alimentaires, comme les bouteilles de lait, dans la composition de flacons cosmétiques, phytosanitaires ou de colorants :
- le PET multicouches, qui comporte des couches de polymères autres que le PET ou une couche de PET recyclé entre des couches de PET vierge (c'est-à-dire PET n'ayant pas subi de recyclage), ou un film d'aluminium par exemple. Le PET multicouches est utilisé après thermoformage pour faire des emballages tels que des barquettes.

Les filières de collecte permettant d'alimenter les filières de recyclage sont structurées différemment en fonction des pays. Elles évoluent de manière à maximiser la quantité de plastique valorisé dans les déchets en fonction de la nature et de la quantité des flux et des technologies de tri. La filière de recyclage de ces flux est en général constituée d'une première étape de conditionnement sous forme de paillettes au cours de laquelle des balles d'emballage brut sont lavées, purifiées et triées, broyées puis de nouveau purifiées et triées pour produire un flux de paillettes contenant en général moins de 1% massique d'impuretés « macroscopiques » (verre, métaux, autres plastiques, bois, papier carton, éléments minéraux), préférentiellement moins de 0,2% d'impuretés « macroscopiques » et encore plus préférentiellement moins de 0,05%.

Les paillettes de PET clair peuvent ensuite subir une étape d'extrusion-filtration permettant de produire des extrudés qui sont ensuite réutilisables en mélange avec du PET vierge pour faire de nouveaux produits (bouteilles, fibres, films). Une étape de polymérisation sous vide à l'état solide (connu sous l'acronyme SSP) est nécessaire pour les usages alimentaires. Ce type de recyclage est appelé recyclage mécanique.

Les paillettes de PET foncé (ou coloré) sont également recyclables mécaniquement. Cependant, la coloration des extrudés formés à partir des flux colorés limite les usages : le PET foncé est le plus souvent utilisé pour produire des fibres ou des lanières d'emballage. Les débouchés sont donc plus limités par rapport à ceux du PET clair.

La présence de PET opaque contenant des pigments à des teneurs importantes, dans le PET à recycler, pose des problèmes aux recycleurs car le PET opaque altère les propriétés mécaniques du PET recyclé. Le PET opaque est actuellement collecté avec le PET coloré et se retrouve dans le flux de PET coloré. Compte tenu du développement des usages du PET opaque, les teneurs en PET opaque dans le flux de PET coloré à recycler sont actuellement comprises entre 5-20% poids et ont tendance à augmenter encore. D'ici quelques années, il sera possible d'atteindre des teneurs en PET opaque dans le flux de PET coloré supérieures à 20-30% poids. Or il a été montré qu'au-delà de 10-15% de PET opaque dans les flux de PET coloré, les propriétés mécaniques du PET recyclé sont altérées (cf. *« Impact du développement du PET opaque blanc sur le recyclage des emballages en PET* », note préliminaire du COTREP du 5/12/13) et empêchent le recyclage sous forme de fibres, principal débouché de la filière pour le PET coloré.

Les colorants sont des substances naturelles ou synthétiques, solubles notamment dans la matière polyester et utilisés pour colorer la matière dans laquelle ils sont introduits. Les colorants généralement utilisés sont de différentes natures et contiennent souvent des hétéroatomes de type O et N, et des insaturations conjuguées, comme par exemple des fonctions quinone, methine, azo, ou des molécules comme la pyrazolone et la quinophtalone. Les pigments sont des substances finement divisées, insolubles en particulier dans la matière polyester, utilisés pour colorer et/ou opacifier la matière dans laquelle ils sont introduits. Les principaux pigments utilisés pour colorer et/ou opacifier les polyesters, en particulier le PET, sont des oxydes métalliques comme TiO₂, CoAl₂O₄, Fe₂O₃, des silicates, des polysulfides, et du noir de carbone. Les pigments sont des particules de taille comprise généralement entre 0,1 et 10 µm, et en majorité entre 0,4 et 0,8 µm. L'élimination totale de ces pigments par filtration, nécessaire pour envisager un recyclage du PET opaque, est techniquement difficile car ils sont extrêmement colmatants.

Le recyclage des PET colorés et opaques est donc extrêmement délicat.

L'amélioration du procédé de polymérisation du PET a également fait l'objet de nombreux travaux. Une partie de ces travaux concernent l'amélioration de la phase de préparation du mélange des monomères, provenant ou pas de la filière de récyclage.

En particulier, la demande de brevet MX 2007/004429 divulgue la production d'un polyester de bonne qualité, comprenant un procédé de dépolymérisation par glycolyse à pression atmosphérique de paillettes de PET en présence d'éthylène glycol dans une base de bis(2-hydroxyéthyl) téréphtalate (BHET). Le produit intermédiaire obtenu à l'issue de l'étape de dépolymérisation est filtré sur un système de fritté pour retenir des particules d'au moins 25µm avant d'être introduit dans le réacteur de polymérisation, pour obtenir un polyester de bonne qualité. Cependant, le document MX 2007/004429 ne divulgue pas l'ajout d'acide téréphtalique avec son intermédiaire dans le réacteur de polymérisation.

La demande de brevet US 2006/0074136 décrit un procédé de dépolymérisation par glycolyse de PET coloré, en particulier issu de la récupération de bouteilles de PET colorées vertes. Le flux de BHET obtenu à l'issue l'étape de glycolyse est purifié sur charbon actif pour séparer certains colorants, comme les colorants bleu, puis par extraction des colorants résiduels, comme les colorants jaune, par un alcool ou par l'eau. Le BHET qui cristallise dans le solvant d'extraction est alors séparé, dans le but de pouvoir être utilisé dans un procédé de polymérisation de PET. Dans la demande de brevet US 2015/0105532, le PET post-consommation comprenant un mélange différents PET colorés comme le PET clair, le PET bleu, le PET vert et/ou le PET ambré, est dépolymérisé par glycolyse en présence d'un catalyseur amine et d'alcool. Le monomère diester alors obtenu peut être purifié par filtration, échange d'ions et/ou passage sur charbon actif, avant d'être cristallisé et récupéré par filtration pour pouvoir être polymérisé et ainsi reformer un polyester. Ces deux demandes de brevets ne détaillent cependant pas les étapes du procédé de (re)polymérisation.

La demande de brevet WO 2017/006217 divulgue le procédé de préparation d'un polyéthylène téréphtalate glycol modifié (r-PETG) comprenant une étape de dépolymérisation d'un PET en présence d'un mélange de monoéthylène glycol (MEG) et de neopentyl glycol, suivie directement d'une étape de polymérisation de l'effluent de réaction. La demande de brevet FR 3053691 décrit un procédé de dépolymérisation d'une charge polyester comprenant en particulier de 0,1 à 10% poids de pigments, par glycolyse en présence d'éthylène glycol. Un effluent de monomères bis-(2-hydroxyethyl) téréphtalate (BHET), obtenu après des étapes particulières de séparation et de purification, peut alimenter une étape de polymérisation en vue de produire du PET, sans qu'aucune condition ne soit préciser.

Le brevet JP3715812 décrit l'obtention de BHET raffiné à partir de PET, le BHET obtenu pouvant être utilisé comme matière première dans un procédé de production de produits en matière plastique. Parallèlement, le brevet EP 1 120 394 divulgue l'utilisation éventuelle comme matière première pour la re-production d'un polyester de haute qualité de bis-(2-hydroxyethyl) téréphtalate de haute pureté. Pour cela, le brevet EP 1 120 394 décrit plus particulièrement un procédé de dépolymérisation d'un polyester sans détailler les étapes aval de polymérisation.

Le brevet US 4,001,187 divulgue des procédé de production de PET de haute qualité, comprenant une étape d'alimentation en continue d'éthylène glycol et d'acide téréphtalique dans le milieu d'estérification comprenant du bis(2-hydroxyéthyl)téréphtalate, les quantités d'acide et de diol introduites étant fonction d'un paramètre lié à la viscosité du mélange (« slurry »). Le brevet US 6,815,525 divulgue un procédé de production de PET, comprenant une étape d'alimentation en continue d'éthylène glycol recyclé, dans le milieu réactionnel. Le brevet US 4,334,090 détaille quant à lui un procédé de préparation du slurry dont la viscosité est améliorée par incorporation de cristaux d'acide téréphtalique pré-traités par attrition. Aucun de ces documents ne propose un procédé de production de polyester avec une amélioration du mélange de monomères acide téréphtalique et diol, permettant de diminuer le taux de solide dudit mélange et donc de faciliter les opérations ultérieures notamment de transport et de réduire la consommation en matières premières, en particulier en diol.

### Résumé de l'invention

L'invention a pour objet un procédé de production d'un polyester téréphtalate, comprenant :
a) une étape de préparation d'une charge d'estérification comprenant au moins une section de mélange alimentée en au moins une charge acide téréphtalique et une charge monomère diester, les quantités en au moins ladite charge acide téréphtalique et ladite charge monomère diester, introduites dans ladite section de mélange dans ledit mélange étant ajustées de sorte que le rapport du nombre total de moles de motifs diol de formule - [C₍ₙ₊₁₎H₍₂ₙ₊₂₎O₂]-, n étant un nombre entier supérieur ou égal à 1, introduits dans ladite section de mélange, par rapport au nombre total de moles de motifs téréphtalate de formule -[CO-(C₆H₄)-CO]-, introduits dans ladite section de mélange, est compris entre 1,0 et 2,0, ladite section de mélange étant opérée à une température comprise entre 25 et 250°C et à une pression supérieure ou égale à 0,1 MPa,
b) une étape d'estérification de ladite charge d'estérification issue de l'étape a), pour produire au moins un effluent réactionnel et un effluent aqueux, ladite étape d'estérification comprenant au moins une section réactionnelle opérée à une température entre 150 et 400°C, à une pression entre 0,05 et 1 MPa, et avec un temps de séjour entre 1 et 10 h, et au moins une section de séparation,
c) une étape de polycondensation dudit effluent réactionnel obtenu à l'étape b) pour obtenir au moins ledit polyester téréphtalate et un effluent diol, ladite étape comprenant au moins une section réactionnelle comprenant au moins un réacteur dans lequel est mise en oeuvre la polycondensation et étant opérée à une température entre 200 et 400°C, à une pression entre 0,0001 et 0,1 MPa, avec un temps de séjour entre 0,1 et 5 h, ladite section réactionnelle comprenant également au moins un soutirage d'un effluent diol,
d) une étape de traitement des diols, comprenant une section de récupération alimentée au moins par tout ou partie de l'effluent diol issu de l'étape c), pour obtenir un effluent diol à traiter et une section de purification dudit effluent diol à traiter pour obtenir un flux diol purifié. De préférence, la présente invention concerne un procédé de production d'un polyester téréphtalate à partir d'au moins une charge polyester à recycler consistant en les étapes a), b), c), et d), décrites ci-dessus.

Un intérêt de l'invention réside dans la préparation optimisée de la charge monomérique. En effet, la présente invention permet la substitution au moins en partie des charges monomères, c'est-à-dire de l'acide téréphtalique et du diol, des procédés classiques de production des polyesters téréphtaliques, par un composé diester téréphtalique.

Ainsi, la présente invention a pour avantage de préparer une charge monomérique présentant un taux de solide diminué par rapport aux mélanges des charges monomères des procédés classiques de production de polyester téréphtalate dans lesquels aucun monomère diester n'est incorporé, le taux de solide étant un taux volumique de solide défini , selon l'invention, comme le ratio du volume de solide par le volume totale de la charge monomérique diphasique (c'est-à-dire de la charge d'estérification selon l'invention). La présente invention permet ainsi de faciliter les opérations de préparation et de transports du mélange diphasique notamment au cours du procédé de production du polyester.

Un autre avantage de la présente invention est la diminution de la quantité de monomère diol introduite dans le procédé de production de polyester par rapport aux procédés classiques de production de polyester, tout en conservant l'opérabilité de l'étape de préparation, c'est-à-dire sans dégrader les conditions de préparation ni la qualité du mélange des charges monomères. La quantité de diol introduit en excès dans le procédé étant diminuée, la quantité de diol récupérée, traitée et recyclée en fin de polymérisation est donc diminuée, induisant de fait, outre la réduction de consommation de matière première diol, une réduction de la consommation énergétique du procédé.

Par ailleurs, lorsque le monomère diester incorporé dans le mélange est avantageusement un intermédiaire diester liquide obtenu à l'issue d'un procédé de dépolymérisation d'un polyester, ledit intermédiaire répondant aux spécifications du procédé de polymérisation est incorporé directement dans le mélange de l'étape a), sans étape supplémentaire de purification et/ou de conditionnement intermédiaire (par exemple une étape de solidification du diester obtenu par dépolymérisation du polyester), limitant ainsi entre autres des consommations d'énergies importantes et répondant ainsi à une attente en matière écologique de la société.

### Description des modes de réalisation

L'invention concerne un procédé de production d'un polyester téréphtalate comprenant notamment une étape de préparation d'une charge de polymérisation particulière.

Selon l'invention, les termes « polyester » et « polyester téréphtalate » sont interchangeables et désignent un polyalkylène téréphtalate. Très classiquement, un polyalkylène téréphtalate est le résultat de la polycondensation d'un monomère diol (ou glycol) avec un monomère acide téréphtalique (ou diméthyle téréphtalate). Le polyester téréphtalate selon l'invention est, en particulier, le polyéthylène téréphtalate (PET), le polybutylène téréphtalate (PBT), le polytriméthylène téréphtalate (PTT) ou tout autre polymère dont le motif de répétition de la chaine principale contient une fonction ester et un cycle aromatique issu de l'acide téréphtalique (ou d'un de ses esters, en particulier du téréphtalate de diméthyle). Selon l'invention, le polyester téréphtalate préféré est le polyéthylène téréphtalate ou poly(téréphtalate d'éthylène), appelé encore simplement PET, dont le motif élémentaire de répétition présente la formule suivante :

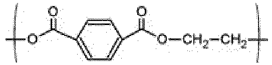

Classiquement, le PET est obtenu par polycondensation de l'acide téréphtalique (PTA), ou du diméthyle téréphtalate (DMT), avec l'éthylène glycol.

Selon l'invention, l'expression « à recycler » qualifie toute matière, en particulier comprenant du polyester, issue des filières de collecte et de tri des déchets plastiques. Par opposition, un polyester vierge est uniquement issu de la polymérisation de charges monomères comprenant au moins un acide dicarboxylique (par exemple l'acide téréphtalique, PTA) ou un ester dicarboxylate (par exemple le téréphtalate de diméthyle, DMT) et au moins un composé de la famille des diols ou glycols (par exemple l'éthylène glycol).

Selon l'invention, le terme « monomère diester » désigne un composé ester téréphtalate de formule chimique HOC₍ₘ₊₁₎H₍₂ₘ₊₂₎-CO₂-(C₆H₄)-CO₂-C₍ₙ₊₁₎H₍₂ₙ₊₂₎OH, dans laquelle : -(C₆H₄)-représente un cycle aromatique ; n et m sont des nombres entiers, identiques ou différents, de préférence identiques (c'est-à-dire n = m), et supérieurs ou égaux à 1, de préférence entre compris 1 et 5, de manière préférée compris entre 1 et 3. Une molécule de monomère diester correspond à un composé qui résulterait de l'estérification d'une molécule d'acide téréphtalique HOOC-(C₆H₄)-COOH (où -(-(C₆H₄)- représentant un cycle aromatique) avec deux molécules d'au moins un diol (ou glycol), plus particulièrement avec une molécule d'un diol de formule chimique HO-C₍ₙ₊₁₎H₍₂ₙ₊₂₎-OH et une molécule d'un diol de formule chimique HO-C₍ₘ₊₁₎H₍₂ₘ₊₂₎-OH. Le monomère diester préféré est le bis(2-hydroxyéthyl) téréphtalate (BHET).

Selon l'invention, la « charge monomère diester » comprend un monomère diester comme défini ci-dessus. La « charge monomère diester » selon l'invention peut également comprendre au moins un diol (ou glycol), de préférence correspondant au(x) motif(s) diol contenu dans ledit monomère diester de ladite charge. Avantageusement, ladite charge monomère diester comprend de préférence au moins 10% poids de monomère diester, préférentiellement au moins 20 % poids.

Par « colorant », on entend une substance soluble dans la matière polyester et utilisée pour la colorer. Le colorant peut être d'origine naturelle ou synthétique.

Par « pigment », plus particulièrement pigment colorant et/ou opacifiant, on entend une substance finement divisée, insoluble dans la matière polyester. Les pigments sont sous forme de particules de taille comprise généralement entre 0,1 et 10 µm, et en majorité entre 0,4 et 0,8 µm. Ils sont souvent de nature minérale. Classiquement, les pigments, notamment opacifiants, utilisés sont des oxydes métalliques comme TiO₂, CoAl₂O₄, Fe₂O₃, des silicates, des polysulfides, et du noir de carbone.

Selon la présente invention, l'expression « compris entre ... et ... » signifie que les valeurs limites de l'intervalle sont incluses dans la gamme de valeurs décrite. Si tel n'était pas le cas et que les valeurs limites n'étaient pas incluses dans la gamme décrite, une telle précision sera apportée par la présente invention.

### Charges

Conformément à l'invention, ledit procédé est alimenté en au moins une charge acide téréphtalique et une charge monomère diester.

La charge acide téréphtalique est avantageusement sous forme de poudre, c'est-à-dire sous forme de particules solides d'acide téréphtalique. Les particules d'acide téréphtalique incorporées dans le mélange de monomères présentent, de préférence, un diamètre moyen de préférence compris entre 1 et 1000 µm, en particulier entre 30 et 500 µm et notamment entre 80 et 200 µm. Le diamètre moyen des particules d'acide téréphtalique est déterminé par toute méthode d'analyse granulométrique connue de l'Homme du métier, comme par exemple par diffraction laser ou par tamisage, de préférence par tamisage sur une colonne de tamis adaptés selon une technique connue de l'Homme du métier.

L'acide téréphtalique de la charge acide téréphtalique peut avantageusement être produit par oxydation du para-xylène ou par dépolymérisation de polyesters ou par tout autre procédé permettant d'obtenir une charge acide téréphtalique avec les spécifications requises par les procédés de polymérisation. L'acide téréphtalique peut être issu de sources hydrocarbures fossiles ou de la biomasse.

Selon l'invention, la charge monomère diester comprend un monomère diester, comme défini plus haut, correspondant à un composé diester téréphtalate de formule chimique HOC₍ₘ₊₁₎H₍₂ₘ₊₂₎-CO₂-(C₆H₄)-CO₂-C₍ₙ₊₁₎H₍₂ₙ₊₂₎OH, dans laquelle : -(C₆H₄)- représente un cycle aromatique ; n et m sont des nombres entiers, identiques ou différents, de préférence identiques (c'est-à-dire n = m), et supérieurs ou égaux à 1, de préférence entre compris 1 et 5, de manière préférée compris entre 1 et 3. Le monomère diester comprend, de préférence, les motifs élémentaires de répétition du polyester téréphtalate produit par le procédé selon l'invention. Très préférentiellement, la charge monomère diester comprend le bis(2-hydroxyéthyl) téréphtalate (BHET), comme monomère diester. La charge monomère diester selon l'invention peut également comprendre au moins un diol, de préférence correspondant au(x) motif(s) diol contenu dans ledit monomère diester de ladite charge. Ladite charge monomère diester comprend de préférence au moins 10% poids de monomère diester, préférentiellement au moins 20% poids.

Selon l'invention, la charge monomère diester peut être sous forme liquide ou sous forme solide, de préférence la charge monomère diester est sous forme liquide.

Dans un mode de réalisation avantageux de l'invention, ladite charge monomère diester de l'étape a) comprend au moins une fraction, de préférence comprend l'intégralité, d'un effluent diester purifié, obtenu à l'issue d'un procédé de dépolymérisation d'une charge polyester à recycler, en particulier obtenu à l'issue des procédés de dépolymérisation d'une charge polyester à recycler des brevets JP 3715812 et FR 3053691, et de la demande WO 01/10812.

Avantageusement, ledit effluent diester purifié est obtenu par un procédé de dépolymérisation d'une charge polyester à recycler comprenant au moins les étapes suivantes :
i) une étape de dépolymérisation comprenant au moins une section réactionnelle alimentée en ladite charge polyester à recycler et en un flux glycol, pour obtenir un effluent réactionnel de dépolymérisation,
ii) une étape de séparation-purification comprenant une section de séparation pour obtenir un effluent glycol et au moins une section de purification pour obtenir un effluent diester purifié, au moins une fraction dudit effluent diester purifié obtenu à l'étape ii) est envoyé vers ladite étape a).

Dans ce mode de réalisation, ladite charge polyester à recycler est issue des filières de collecte et de tri des déchets, en particulier des déchets plastiques. De préférence, ladite charge polyester à recycler est une charge polyéthylène téréphtalate PET à recycler.

La charge polyester à recycler qui alimente l'étape de dépolymérisation i) peut être sous forme de paillettes (ou flakes selon le terme anglais), dont la plus grande longueur est inférieure à 10 cm, préférentiellement comprise entre 5 et 25 mm, ou sous forme de solide micronisé c'est-à-dire sous de particules de préférence ayant une taille comprise entre 10 micron et 1 mm. La charge polyester à recycler comprend de préférence moins de 2% poids, préférentiellement moins de 1 % poids d'impuretés « macro » comme des déchets en verre, en métal, en plastique autre que polyester téréphtalate, en bois, papier carton, éléments minéraux. Ladite charge polyester à recycler peut également être sous forme de fibres, telles que des fibres textiles, éventuellement prétraitées pour éliminer des fibres de coton, de polyamide, ou tout autre fibre textile autre que polyester, ou telles que des fibres de pneus, éventuellement prétraitées pour éliminer notamment des fibres polyamide ou des résidus de caoutchouc ou de polybutadiène. Ladite charge polyester à recycler, qui alimente l'étape de dépolymérisation i), contient avantageusement plus de 50 % masse de polyalkylène téréphtalate, de préférence plus de 70% poids, de manière préférée plus 90 % poids de polyalkylène téréphtalate.

Ladite charge polyester à recycler peut être obtenues à partir de déchets de polyester téréphtalate, de préférence de PET, clair, coloré, opaque, foncé et/ou multicouche. Elle comprend avantageusement au moins un polyester téréphtalate, de préférence PET, opaque, foncé ou multicouche. De manière préférée, elle comprend au moins 10% poids de polyester téréphtalate opaque, de préférence de PET opaque, de manière très préférée au moins 15% poids de polyester téréphtalate opaque, de préférence de PET. Ladite charge polyester à recycler peut contenir jusqu'à 10% poids de pigments, en particulier entre 0,1% et 10% poids de pigments, notamment entre 0,1 et 5% poids de pigments de pigments, et/ou jusqu'à 1% poids de colorants, en particulier entre 0,05% et 1% poids de colorants, notamment entre 0,05 et 0,2% poids de colorants.

Ladite charge polyester à recycler peut également contenir des éléments utilisés comme catalyseur de polymérisation et/ou comme agents stabilisants dans les procédés de production de polyester, comme l'antimoine, le titane, l'étain.

Avantageusement, l'étape i) de dépolymérisation met en œuvre, dans la section réactionnelle, une réaction de glycolyse du polyalkylène téréphtalate de ladite charge polyester à recycler, en présence dudit flux glycol, dans un ou plusieurs réacteurs. Ledit flux glycol comprend, de préférence, un monomère diol qui correspond au motif diol dans la composition du motif élémentaire de répétition du polyester téréphtalate produit par le procédé de production selon l'invention. De préférence ledit flux glycol est un flux d'éthylène glycol. Avantageusement, ledit flux glycol qui alimente l'étape i) de dépolymérisation comprend, de préférence consiste en, au moins une fraction dudit flux diol purifié obtenu à l'issue de l'étape d) du procédé de production du polyester téréphtalate selon l'invention. Ladite section réactionnelle de l'étape i) de dépolymérisation est opérée à une température comprise entre 150 et 400°C, de préférence entre 180 et 300°C, de manière préférée entre 200°C et 280°C, à une pression opératoire d'au moins 0,1 MPa, préférentiellement au moins 0,4 MPa, et avec un temps de séjour par réacteur compris entre 0,05 et 10 h de préférence entre 0,1 et 6 h, de manière préférée entre 0,5 et 4 h, ledit temps de séjour par réacteur étant défini comme le rapport du volume liquide du réacteur par le débit volumique du flux qui alimente ledit réacteur. Le flux glycol alimente la section réactionnelle de telle sorte que la quantité du composé glycol contenu dans dudit flux glycol est ajustée de 1 à 20 moles de diol dudit flux glycol par mole de motif élémentaire de répétition du polyester contenu dans ladite charge polyester à recycler, de préférence de 3 à 10 moles de diol dudit flux glycol par mole de diester dans ladite charge polyester à recycler.

La réaction de dépolymérisation peut être réalisée avec ou sans adjonction d'un catalyseur. Lorsque la réaction de dépolymérisation est réalisée après adjonction d'un catalyseur, ce dernier peut être homogène ou hétérogène et choisi parmi les catalyseurs d'estérification connus de l'Homme du métier tels que les complexes oxydes et sels d'antimoine, d'étain, de titane, les alkoxydes de métaux des groupes (I) et (IV) de la classification périodique des éléments, les peroxydes organiques, les oxydes métalliques acido-basiques. De préférence, la réaction de dépolymérisation est réalisée sans adjonction de catalyseur.

La réaction de dépolymérisation peut également être avantageusement réalisée en présence d'un agent adsorbant solide sous forme de poudre ou mis en forme, dont la fonction est de capter au moins une partie des impuretés, en particulier des impuretés colorées, soulageant ainsi la phase de purification de l'étape ii). Ledit agent adsorbant solide est en particulier un charbon actif.

L'effluent réactionnel de dépolymérisation obtenu à l'issue de l'étape i) de dépolymérisation comprend un mélange de monomères diesters, d'oligomères comprenant entre 1 et 5, de préférence entre 1 et 3, motifs élémentaires de formule -[O-CO-(C₆H₄)-CO-O- C₍ₙ₊₁₎H₍₂ₙ₊₂₎]-, avec n un nombre entier compris entre 1 et 5, de préférence entre 1 et 5, de composés diols, d'impuretés éventuellement présentes dans ladite charge polyester et de composés éventuellement produits à l'issu de réactions secondaires comme par exemple des réactions d'éthérification ou de dégradation. Les composés diols sont avantageusement les monomères et co-monomères diols entrant dans la composition de la charge polyester à recycler et libérés à l'issue de la réaction de dépolymérisation et ceux non réagis issus du flux glycol alimentant l'étape i) de dépolymérisation. Ledit effluent réactionnel de dépolymérisation peut également contenir des polyesters non converti et autres polymères. L'effluent réactionnel de dépolymérisation obtenu à l'issue de l'étape i) de dépolymérisation alimente l'étape ii) de séparation-purification qui comprend au moins une section de séparation d'un effluent glycol et au moins une section de purification pour obtenir un effluent diester purifié.

Lesdites sections de séparation et de purification peuvent être dans un ordre ou dans un autre l'une par rapport à l'autre. Lesdites sections de séparation et de purification peuvent aussi être interconnectées lorsque l'étape ii) comprend une section de purification de l'effluent réactionnel de dépolymérisation pour obtenir un effluent réactionnel de dépolymérisation purifié, une section de séparation dudit effluent réactionnel de dépolymérisation purifié pour obtenir un effluent glycol et un effluent diester, et une section de purification dudit effluent diester pour obtenir un effluent diester purifié.

Dans un mode de réalisation préféré, l'étape ii) comprend une section de séparation dudit effluent réactionnel de dépolymérisation pour obtenir un effluent glycol, comprenant avantageusement le diol du flux glycol de l'étape i) n'ayant pas réagi, et un effluent diester, et une section de purification dudit effluent diester pour obtenir un effluent diester purifié. Ledit effluent glycol obtenu en sortie de section de séparation de l'étape ii) comprend avantageusement plus de 50% poids, de préférence plus de 70% poids, de manière préférée plus de 90% poids de diols. Ladite section de séparation permet avantageusement de récupérer le diol du flux glycol de l'étape i) n'ayant pas réagi. Ledit effluent diester obtenu en sortie de section de séparation est de préférence sous forme liquide et comprend avantageusement plus de 10% poids, de préférence plus de 25% poids, de manière préférée plus de 50% poids de monomères et oligomères diesters.

Avantageusement, ladite section de séparation comprend un ou plusieurs dispositifs de séparation, pour permettre la récupération d'un effluent enrichi en diols (l'effluent glycol), et éventuellement d'un effluent enrichi en impuretés légères et d'un effluent enrichi en impuretés lourdes. Toute méthode de séparation physique, chimique ou physico-chimique connu de l'Homme du métier peut être utilisé, comme par exemple la séparation gaz-liquide, distillation, évaporation, extraction par un solvant couplée ou non avec une réaction chimique, cristallisation suivie d'une filtration ou centrifugation ou une association desdites méthodes de séparation. De préférence, ladite section de séparation comprend une succession de séparations gaz-liquide, préférence 1 à 5 séparations gaz-liquide, opérées à une température comprise entre 100 et 250°C, de préférence entre 110 et 220°C, de manière préférée entre 120 et 210 °C, et à une pression comprise entre 0,00001 et 0,2 MPa, de préférence entre 0,00004 et 0,15 MPa, de manière préférée entre 0,00004 et 0,1 MPa. De préférence, tout ou partie dudit effluent glycol récupéré à l'issu de l'étape ii) est avantageusement envoyé à l'étape d) de traitement du procédé selon l'invention.

Tout ou partie dudit effluent glycol récupéré à l'issu de l'étape ii) peut être pré-purifié dans une section de pré-purification des diols comprise dans l'étape ii) pour éliminer une partie des impuretés entrainées avec ledit effluent glycol comme par exemple des colorants, pigments ou autres particules solides. La section de pré-purification alimentée par tout ou partie dudit effluent glycol peut comprendre, de manière non exhaustive, une adsorption sur solide (par exemple sur charbon actif) et un système de filtration. Au moins une fraction dudit effluent glycol pré-purifié peut être directement recyclée vers l'étape i) de dépolymérisation. Une séparation des différents diols éventuellement compris dans ledit effluent glycol peut être mise en œuvre dans ladite section de pré-purification.

La phase de purification de l'effluent diester consiste à séparer au moins un effluent monomère diester purifié, de tout ou partie des composés suivants issus de l'étape i de dépolymérisation : oligomères diesters, polyester éventuellement non converti, impuretés éventuellement présentes dans la charge polyester à recycler telles que d'autres polymères, des pigments, des colorants, des catalyseurs de polymérisation ou tout autre composé inorganique composant ladite charge polyester à recycler ou formé lors de l'étape i) de dépolymérisation, tout en minimisant la perte en monomère diester.

La purification dudit effluent diester est avantageusement réalisée par toute méthode physique, chimique ou physico-chimique connu de l'Homme du métier, permettant de récupérer un effluent monomère diester purifié avec un rendement en monomère diester supérieur ou égal à 50 % poids, de préférence supérieur ou égal à 70 % poids, de manière préférée supérieur ou égal à 80% poids. Par rendement, on entend la quantité de monomère diester dans ledit flux de monomère diester purifié rapportée à la quantité totale de monomère diester introduite dans la section de purification. De préférence, ledit effluent monomère diester purifié est exempt de colorants ou d'impuretés inorganiques tel que des pigments, des catalyseurs de dépolymérisation et des ions. De préférence, le monomère diester purifié comprend les molécules de monomère diester et éventuellement des oligomères dudit diester avec une degré de polymérisation compris entre 2 et 5.

La purification dudit effluent diester (et/ou dudit effluent réactionnel de dépolymérisation) met avantageusement en œuvre une ou plusieurs opérations de purification, comme la filtration, l'évaporation, la distillation, la séparation par membrane, la précipitation ou la cristallisation, l'adsorption sur une masse de captation, le traitement sur résine échangeuse d'ions ou l'extraction par un solvant. Par exemple, dans le brevet EP 0865464, la purification de l'effluent diester comprend une succession d'opération de dissolution dans un solvant chaud puis précipitation et filtration pour séparer les impuretés de taille supérieure à 50µm et la séparation des monomères diester et oligomères dans un évaporateur à film raclé (thin film evaporator selon le terme anglais). Eventuellement, la section de purification de l'étape ii) peut comprendre au moins deux opérations (ou deux phases) de purification :
- une première phase de purification qui permet la séparation d'impuretés insolubles dans ledit effluent diester (et/ou dudit effluent réactionnel de dépolymérisation), ou devenues insolubles après refroidissement ou évaporation partielle dudit effluent ou ajout d'un tiers corps tel que par exemple un agent floculant ou solvant favorisant la précipitation ;
- une seconde phase de purification qui permet la séparation d'impuretés solubles dans ledit effluent diester (et/ou dudit effluent réactionnel de dépolymérisation) ou devenues solubles après chauffage ou ajout d'un solvant.

De manière préférée, la purification dudit effluent diester (et/ou dudit effluent réactionnel de dépolymérisation) met en œuvre une section de séparation comprenant un système d'évaporation à film tombant ou à film raclé, ou une distillation à court trajet à film tombant ou à film raclé, ou une succession de plusieurs évaporations et/ou distillations court trajet à film tombant ou film raclé, opérée à une température inférieure ou égale à 250°C, de préférence inférieure ou égale à 230°C, préférentiellement inférieure ou égale à 200°C, et à une pression inférieure ou égale à 0,001 MPa, de préférence inférieure ou égale à 0,0001 MPa, de manière préférée inférieure ou égale à 0,00005 MPa, puis une section de décoloration opérée à une température entre 100 et 250°C, de préférence entre 110 et 200°C, et de manière préférée entre 120 et 180°C, et à une pression entre 0,1 et 1,0 MPa, de préférence entre 0,2 et 0,8 MPa, et de manière préférée entre 0,3 et 0,5 MPa, en présence d'un adsorbant, de préférence un charbon actif.

Avantageusement, l'effluent diester purifié obtenu à l'issue de l'étape ii) comprend au moins 10 % poids de monomère diester, de préférence au moins 20 % poids de monomère diester. Il contient de préférence moins de 1% poids, de préférence moins de 0,1% poids, des pigments introduits dans le procédé avec la charge polyester à recycler et moins de 10% poids, de préférence moins de 1% poids, des colorants introduits dans le procédé avec la charge polyester à recycler.

Ladite étape ii) peut également produire un effluent d'impuretés ester composé d'oligomères et éventuellement de polymères non convertis à l'étape i) de dépolymérisation. Ledit effluent d'impuretés ester peut avantageusement être tout ou partie recyclé vers l'étape i) ou purgé et envoyé vers un système d'incinération. Le cas échéant, ladite fraction dudit effluent d'impuretés ester recyclée vers l'étape i) peut subir au moins une opération de séparation, de préférence une opération de filtration, de manière à réduire la quantité de pigments et/ou autres impuretés solides éventuellement présents dans ledit effluent d'impuretés ester. Eventuellement, tout ou partie d'au moins une fraction de l'effluent glycol issue de l'étape ii) ou de l'étape d) du procédé selon l'invention peut avantageusement être mélangée avec ladite fraction de l'effluent d'impuretés ester recyclée de manière à diminuer la viscosité de ladite fraction dudit effluent d'impuretés ester et faciliter son transport vers l'étape i) et éventuellement son traitement dans une étape optionnelle de filtration.

De préférence, l'effluent diester purifié est récupéré à l'issue de l'étape ii) sous forme liquide ou sous forme solide, de préférence sous forme liquide.

Avantageusement, au moins une fraction dudit effluent diester purifié obtenu à l'étape ii) est envoyée vers l'étape a) du procédé de production d'un polyester téréphtalate selon l'invention.

### Etape a) de préparation de la charge d'estérification

Conformément à l'invention, le procédé de production d'un polyester téréphtalate comprend une étape a) de préparation d'une charge d'estérification. Ladite étape a) comprend au moins une section de mélange alimentée en au moins une charge acide téréphtalique, et une charge monomère diester.

La charge d'estérification, selon l'invention, qui est obtenue à l'issue de l'étape a), est un mélange diphasique homogène, comprenant au moins de l'acide téréphtalique, un monomère diester et éventuellement un diol (ou glycol) de formule chimique HO-C₍ₙ₊₁₎H₍₂ₙ₊₂₎-OH, n étant un nombre entier supérieur ou égal à 1, de préférence entre compris 1 et 5, de manière préférée compris entre 1 et 3. Par « diphasique », on entend avantageusement une suspension d'une phase solide dans une phase liquide ou pâteuse. Par « homogène », il faut entendre que la phase solide, en suspension dans la phase liquide ou pâteuse, est répartie dans l'ensemble de la phase liquide ou pâteuse de façon homogène. Plus particulièrement, la charge d'estérification selon l'invention est un mélange de particules solides d'acide téréphtalique, de diamètre typiquement compris entre 1 et 1000 µm, notamment entre 80 et 300 µm, réparties de façon homogène dans une phase liquide ou pâteuse comprenant les monomères diol et les monomères diester.

Avantageusement, les quantités des charges monomères, c'est-à-dire les quantité en au moins la charge acide téréphtalique et la charge monomère diester, introduites dans ladite section de mélange, sont ajustées de sorte que le rapport du nombre total de moles de motifs diol de formule -[C₍ₙ₊₁₎H₍₂ₙ₊₂₎O₂]-, n étant un nombre entier supérieur ou égal à 1, introduits dans ladite section de mélange, par rapport au nombre total de moles de motifs téréphtalate de formule -[CO-(C₆H₄)-CO]-, introduits dans ladite section de mélange, est compris entre 1,0 et 2,0, de préférence entre 1,0 et 1,5, de manière préférée entre 1,0 et 1,3. De préférence, la section de mélange de l'étape a) est alimentée en charge monomère diester sous forme liquide. Lorsque la charge monomère diester destinée à alimenter la section de mélange est sous forme solide, l'étape a) du procédé de l'invention peut éventuellement comprendre une section de conditionnement, située en amont de la section de mélange, pour obtenir une charge monomère diester liquide. Ladite éventuelle section de conditionnement est au moins alimentée en la charge monomère diester sous forme solide et opérée à une température supérieure à la température de liquéfaction de ladite charge monomère diester, de préférence comprise entre 25 et 250°C et à une pression supérieure ou égale à 0,1 MPa. La pression de ladite section de mélange est très avantageusement inférieure ou égale à 5 MPa.

Dans un mode de réalisation préférée de l'invention, ladite section de mélange de ladite étape a) du procédé selon l'invention est en outre alimentée en une charge monomère diol, de préférence comprenant un monomère diol correspondant au(x) motif(s) diol contenu(s) dans le monomère diester de ladite charge monomère diester. Elle comprend majoritairement le monomère diol qui entre dans la composition du motif élémentaire de répétition du polyester téréphtalate produit par le procédé selon l'invention. De préférence, la charge monomère diol comprend au moins 70% mol., préférentiellement au moins 90% mol., de manière très préférée 99,5% mol., d'un monomère diol entant dans la composition du motif unitaire du polyester téréphtalate visé. De manière préférée, la charge monomère diol comprend de l'éthylène glycol. Ladite charge monomère diol est de préférence sous forme liquide.

De préférence, ladite charge en monomère diol peut être, au moins en partie, une fraction du flux diol purifié obtenu à l'étape d) du procédé selon l'invention. Ladite charge en monomère diol peut éventuellement comprendre une source de diol externe.

Lorsqu'une charge en monomère diol est incorporée dans la section de mélange de l'étape a), la quantité en ladite charge diol introduite dans la section de mélange de l'étape a) est ajustée de sorte que le rapport du nombre de motifs diol par rapport au nombre de motifs téréphtalate dans le mélange de l'étape a), comme défini ci-dessus, est compris entre 1,0 et 2,0, de préférence entre 1,0 et 1,5 et de manière préférée entre 1,0 et 1,3.

Avantageusement, la quantité de molécules de monomère diester contenues dans la charge monomère diester introduite dans la section de mélange de l'étape a) représente au moins 5% poids par rapport au poids d'acide téréphtalique (PTA), de préférence au moins 15 % poids.

Une molécule de monomère diester de la charge monomère diester comprend deux motifs diol et un motif téréphtalate. Une molécule d'acide téréphtalique comprend un motif téréphtalate. Une molécule de diol comprend un motif diol. Ainsi, l'incorporation d'une mole de monomère diester, par exemple une mole de bis(2-hydroxyéthyl) téréphtalate (BHET), en mélange avec les charges monomères acide téréphtalique et diol, de préférence un diol correspondant au motif diol contenu dans ledit monomère diester, tel que l'éthylène glycol, permet de substituer une partie de ladite charge acide téréphtalique et tout ou partie de ladite charge diol.

Avantageusement, ladite section de mélange dans l'étape a) du procédé selon l'invention est opérée à une température comprise entre 25 et 250°C, de préférence entre 60 et 200°C, de manière préférée entre 100 et 150°C, et à une pression supérieure ou égale à 0,1 MPa.. La pression de ladite section de mélange est très avantageusement inférieur ou égale à 5 MPa. Un ou plusieurs catalyseurs de polymérisation peu(ven)t, en outre, être incorporé(s) dans le mélange de l'étape a) du procédé selon l'invention.

D'autres composés monomères (ou co-monomères) peuvent également être avantageusement introduits dans le mélange et se retrouver dans la charge d'estérification. De manière non exhaustive, lesdits autres composés monomères peuvent être des acides dicarboxyliques tels que par exemple l'acide iso-phtalique et des diols tels que par exemple le 1,4-dihydroxy-méthylcyclohexane et le diéthylène glycol.

Le procédé selon l'invention, en incorporant dans la charge d'estérification un monomère diester, par exemple des monomères BHET, permet ainsi de substituer une partie de l'acide téréphtalique, qui est un composé sous forme de poudre de particules solides, dans le mélange diphasique des monomères pour la production de polyester. Le taux de solide de ce mélange diphasique peut donc être diminué par rapport aux procédés classiques de production de polyester, facilitant ainsi les opérations industrielles ultérieure, notamment son transport. La présence de monomère diester, en particulier de BHET, peut aussi favoriser une augmentation de la vitesse d'estérification de l'acide téréphtalique. La substitution d'une partie de l'acide téréphtalique et tout ou partie du diol par des monomères diester, dans le procédé selon l'invention, permet également, à taux de solide du mélange de réaction diphasique identique par rapport au celui des procédés classiques de production de polyester, de diminuer la quantité d'éthylène glycol introduit en excès dans le mélange, induisant une réduction des coûts notamment de matières premières mais aussi une réduction conséquente des consommations énergétiques du procédé de production de polyester, du fait d'une quantité fortement diminuée de matière à traiter et recycler.

### Etape b) d'estérification

Conformément à l'invention, le procédé de production d'un polyester téréphtalate comprend une étape b) d'estérification de la charge d'estérification obtenue à l'issue de l'étape a), pour produire au moins un effluent réactionnel et un effluent aqueux.

Ledit effluent réactionnel comprend avantageusement des diesters et des oligomères d'ester. De préférence, les diesters dans ledit effluent réactionnel sont de même nature que ledit monomère diester incorporé dans le mélange de l'étape a). De préférence, les oligomères d'ester dans ledit effluent réactionnel se composent avantageusement des motifs élémentaires correspondant aux motifs élémentaires de répétition du polyester téréphtalate produit par le procédé selon l'invention.

Avantageusement, ladite étape b) d'estérification comprend au moins une section réactionnelle et au moins une section de séparation pour séparer ledit effluent réactionnel et ledit effluent aqueux.

La réaction mise en oeuvre à l'étape b) comprend avantageusement une réaction d'estérification qui consiste en une réaction de condensation d'au moins les groupes hydroxyles (-OH) du monomère diester de la charge monomère diester incorporée à la charge d'estérification à l'étape a), et des monomères diol éventuellement présents dans la charge d'estérification, avec au moins les groupes carboxyles (-COOH) de l'acide téréphtalique de la charge acide téréphtalique incorporée à la charge d'estérification à l'étape a). Cette réaction d'estérification produit des molécules de monomère diester, par exemple du bis(2-hydroxyethyl) téréphtalate (BHET), et des oligomères de diester comprenant avantageusement 2 à 5 motifs téréphtalate. Elle libère également de l'eau. La réaction mise en oeuvre à l'étape b) du procédé selon l'invention comprend également avantageusement des réactions de transestérification consistant en la réaction de condensation de molécules de monomère diester entre elles, libérant ainsi des molécules de diol.

Ladite section réactionnelle est opérée à une température entre 150 et 400°C, de préférence entre 200 et 300°C, à une pression entre 0,05 et 1 MPa, de préférence entre 0,1 et 0,3 MPa, et avec un temps de séjour entre 0,5 et 10 h, de préférence entre 1 et 5 h. Selon l'invention, le temps de séjour dans ladite étape b) d'estérification est défini comme le rapport du volume réactionnel d'un réacteur de ladite section réactionnelle sur le débit volumique du flux liquide sortant dudit réacteur. La réaction d'estérification est avantageusement mise en oeuvre dans un ou plusieurs réacteurs agités en série ou en parallèle, dans un ou plusieurs réacteurs tubulaires en série ou en parallèle ou dans une association de réacteurs agités et tubulaires en série ou en parallèle.

Dans ladite section de séparation de l'étape b), l'eau formée lors de la réaction d'estérification est séparée. Avantageusement, la section réactionnelle comprend également au moins un soutirage d'un effluent soutiré riche en eau et en diol. L'eau est séparée en particulier par différence de volatilité, par exemple par distillation, ou par adsorption à partir de l'effluent soutiré du milieu réactionnel contenant au moins une partie du diol et de l'eau libérée présent dans le milieu réactionnel.

Avantageusement, un catalyseur de polymérisation connu de l'Homme du métier, éventuellement en mélange avec un flux diol, alimente une section de finition de l'étape b) d'estérification. Les catalyseurs de polymérisation sont de manière non exhaustive des catalyseurs à base d'antimoine, de titane, de germanium ou d'aluminium, de l'acétate de zinc, de calcium ou de manganèse.

L'incorporation de la charge monomère diester aux charges monomères du procédé de polymérisation selon l'invention permet de substituer au moins une partie de la charge acide téréphtalique et tout ou partie de la charge diol, permettant de réduire la quantité d'eau formée et donc d'effluent soutiré du milieu réactionnel à traiter. La consommation énergétique s'en trouve avantageusement diminuée.

### Etape c) de polycondensation

Conformément à l'invention, le procédé de production d'un polyester téréphtalate comprend une étape c) de polycondensation de l'effluent réactionnel obtenu à l'étape b), pour obtenir au moins ledit polyester téréphtalate et un effluent diol. Ledit effluent diol comprend au moins un monomère diol correspond avantageusement au motif diol, de formule -[C₍ₙ₊₁₎H₍₂ₙ₊₂₎O₂]-, n étant un nombre entier supérieur ou égal à 1, compris au moins dans le diester de la charge monomère diester qui alimente la section de mélange dans l'étape a) du procédé selon l'invention.

L'étape c) de polycondensation consiste à réaliser une réaction de condensation entre les monomères et oligomères diesters obtenus à l'étape b) d'estérification pour obtenir un polyester avec un degré de polymérisation donné et les propriétés physico-chimiques désirés (par exemple : indice de viscosité, cristallinité, couleur, propriétés mécaniques,...). Ladite réaction de condensation libère des composés diols, éventuellement de l'eau et des co-produits, qu'il convient d'éliminer.

L'étape c) de polycondensation comprend au moins une section réactionnelle comprenant au moins un réacteur dans lequel est mise en oeuvre la polycondensation et au moins un soutirage d'un effluent diol, comprenant avantageusement au moins un monomère correspondant au motif diol de formule -[C₍ₙ₊₁₎H₍₂ₙ₊₂₎O₂]-, n étant un nombre entier supérieur ou égal à 1, compris au moins dans le diester de la charge monomère diester qui alimente la section de mélange dans l'étape a) du procédé selon l'invention.

Avantageusement, ladite section réactionnelle est opérée dans un ou plusieurs réacteurs, fonctionnant en série ou en parallèle, à une température 200 et 400°C, de préférence entre 250 et 300°C, à une pression entre 0,0001 et 0,1 MPa, de préférence entre 0,0004 et 0,01 MPa, avec un temps de séjour entre 0,1 et 5 h, de préférence entre 0,5 et 3 h. Selon l'invention, le temps de séjour dans ladite étape c) de polycondensation est défini comme le rapport du volume réactionnel d'un réacteur de ladite section réactionnelle sur le débit volumique du flux liquide sortant dudit réacteur. La réaction de condensation à l'étape c) de polycondensation peut être réalisée en deux étapes réactionnelles successives, une étape de condensation en phase fondue et suivie d'une étape de post-condensation en phase solide.

Avantageusement, des additifs et catalyseurs de polymérisation peuvent être introduits l'étape c) de polycondensation. De manière non exhaustive, les additifs peuvent comprendre des agents d'inhibition des réactions secondaires d'éthérification comme par exemple des amines (n-butylamine, diisopropylamine ou triethylamine), de l'hydroxyde de sodium ou des hydroxydes organiques ou du carbonate de lithium, des agents stabilisants tel que des phosphites ou des phosphates, et des composés de type polyamides pour réduire la quantité de produit de dégradation comme l'acétaldéhyde. Les catalyseurs de polymérisation couramment utilisés, sont comme par exemple, des catalyseurs à base d'antimoine, de titane, de germanium ou d'aluminium, de l'acétate de zinc, de calcium ou de manganèse. Avantageusement, le soutirage dudit effluent diol est mis en œuvre à l'aide d'un ou plusieurs système(s) de soutirage, avantageusement connecté au(x) réacteur(s) de la section réactionnelle de ladite étape c), et permet de séparer le monomère diol libéré lors de la réaction de condensation et éventuellement l'eau et d'autres co-produits éventuellement libérés au cours de la réaction de condensation. De préférence, l'effluent diol, soutiré du(des) réacteurs de l'étape c), est un effluent gazeux qui est ensuite avantageusement refroidi à une température entre 0 et 100°C et condensé pour obtenir un effluent sous forme liquide, ledit effluent liquide comprenant au moins le monomère diol.

De manière préférée, au moins une fraction de l'effluent comprenant au moins le monomère diol, de préférence sous forme liquide, est envoyé à l'étape d) du procédé selon l'invention.

Avantageusement, ledit effluent comprenant au moins le monomère diol, de préférence sous forme liquide, peut être tout ou partie recyclé directement à l'étape a) de préparation d'une charge d'estérification.

Dans un mode de réalisation très particulier, ledit effluent comprenant au moins le monomère diol, de préférence sous forme liquide, peut être tout ou partie recyclé directement vers l'étape b) d'estérification..

### Etape d) de traitement des diols

Conformément à l'invention, le procédé de production d'un polyester téréphtalate comprend une étape d) de traitement des diols, comprenant une section de récupération alimentée au moins par tout ou partie de l'effluent diol, issu de l'étape c), pour obtenir un effluent diol à traiter, et une section de purification dudit effluent diol à traiter pour obtenir un flux diol purifié.

Avantageusement, ladite section de récupération de l'étape d) est alimentée au moins par tout ou partie de l'effluent diol obtenu à l'étape c), de préférence sous forme liquide. Elle peut être alimentée en outre par tout ou partie de l'effluent glycol issu de la section de séparation de l'étape ii) du procédé de dépolymérisation d'une charge polyester à recycler, dans le cas du mode de réalisation avantageux de l'invention dans lequel la charge monomère diester de l'étape a) comprend au moins une fraction de l'effluent diester purifié, obtenu à l'issue du procédé de dépolymérisation d'une charge polyester à recycler. Eventuellement, ladite section de récupération de l'étape d) du procédé selon l'invention peut également être alimentée par un appoint externe de diol. Avantageusement, la section de récupération peut comprendre une ou plusieurs opérations de filtration des différents flux comprenant au moins le monomère diol.

Un effluent diol à traiter est obtenu à l'issue de la section de récupération de l'étape d) du procédé selon l'invention et est envoyé dans ladite section de purification pour obtenir un flux diol purifié.

Ladite section de purification comprend au moins un système de séparation permettant de mettre en oeuvre toute méthode de séparation physique, physico-chimique ou chimique connue de l'Homme du métier, comme par exemple la séparation gaz-liquide, la distillation, l'adsorption. De préférence, la purification dudit effluent diol à traiter met en oeuvre au moins colonne de distillation, de préférence une série de colonne de distillation, opérée à une température entre 50 et 250°C, de préférence entre 70 et 220°C et à une pression entre 0,001 et 0,2 MPa, de préférence entre 0,01 et 0,1 MPa. De manière préférée, ladite section de purification comprend une phase de séparation des impuretés plus légères que le monomère diol de l'effluent diol à traiter et une phase de séparation des impuretés plus lourdes que le monomère diol de l'effluent diol à traiter, de préférence dans une série de colonnes de distillation.

Avantageusement, ladite étape d) peut également comprendre une section d'élimination des composés organiques volatiles par combustion thermique ou catalytique desdits composés pour éviter leur rejet dans l'environnement. De manière non exhaustive, ladite section de traitement des impuretés comprend une filtration si présence de particules solides et un système de combustion catalytique ou non.

Le procédé selon l'invention permet ainsi d'obtenir un polyester téréphtalate, ayant avantageusement le degré de polymérisation visé et les propriétés physico-chimiques désirées, à partir d'un mélange amélioré des monomères permettant de limiter la consommation en monomère diol et de réduire la quantité de monomère diol éventuellement introduit, en excès, dans le mélange de monomères (c'est-à-dire la charge d'estérification) qui n'a pas été converti. Cette consommation réduite en diol limite ainsi la quantité de diol à recycler et donc réduit de ce fait les consommations énergétiques du procédé.

Le procédé permet également, en substituant un monomère acide et deux monomères diol par un monomère diester, de réduire le taux de solide du mélange monomères de départ de polymérisation, facilitant ainsi les opérations ultérieures, notamment le transport de ce mélange diphasique.

Les exemples suivants illustrent l'invention sans en limiter la portée.

### Exemples

### EXEMPLE 1 - Comparatif

5,5 t/h d'acide téréphtalique (PTA) sont introduits dans un ballon mélangeur équipé d'une agitation mécanique et mélangé à 110°C avec 2,5 t/h d'un flux de monoéthylène glycol (MEG) comprenant 2,13 t/h de MEG provenant d'un bac de stockage et 0,37 t/h de MEG recyclé provenant de la section de purification du MEG.

Les quantités de PTA et MEG introduits correspondent à un ratio molaire PTA/MEG de 1,23. A 110°C, 1 % massique de PTA initialement introduit est solubilisé dans le MEG, et le taux volumique de solide, défini comme le ratio du volume de solide par le volume totale de la pâte (solide + liquide), est de 60,7 % volumique. Le mélange obtenu forme une pâte visqueuse.

Le mélange obtenu est ensuite transférée à l'aide d'une pompe adaptée vers un premier réacteur d'estérification opéré à 260°C, 0,5 MPa avec un temps de séjour de 1,25 h. 1.4 t/h d'un effluent vapeur comprenant 71% poids d'eau et 29% poids de MEG est soutiré et envoyé dans une colonne de reflux pour séparer l'eau formée par la réaction d'estérification et le MEG. Ce dernier est ensuite renvoyé dans le réacteur. Une conversion du PTA de 85% est obtenue dans le premier réacteur.

L'effluent liquide du premier réacteur est envoyé ensuite dans un second réacteur d'estérification opéré à 260°C et 0,2 MPa avec un temps de séjour de 1,25 h. 140 kg/h d'un effluent vapeur comprenant 40% poids d'eau et 60% poids de MEG est soutiré du second réacteur et envoyé à la colonne de reflux. Une conversion du PTA de 92% est atteinte à la sortie du second réacteur.

L'effluent liquide du second réacteur d'estérification est envoyé dans un troisième réacteur opéré à 275°C et 0,033 MPa avec un temps de séjour de 0,5 h qui permet de pousser la conversion du PTA à 95,8% et d'initier la polycondensation. Du trioxyde d'antimoine est ajouté comme catalyseur de polymérisation en entrée du troisième réacteur à hauteur de 220 ppm poids. Un effluent vapeur comprenant 70% poids de MEG, 16,5% poids d'eau, 5,5% poids d'acétaldéhyde, 2,5% poids de diéthylène glycol et 5,5% poids d'oligomères est soutiré du troisième réacteur et partiellement condensé puis envoyé à la section de purification du MEG.

L'effluent liquide du troisième réacteur est envoyé dans un quatrième réacteur (réacteur de polycondensation) opéré à 275°C et 0,0066 MPa avec un temps de séjour de 0,5 h. Un effluent vapeur de composition 60% poids de MEG, 25% poids d'eau, 6% poids d'acétaldéhyde, 3% poids de diéthylène glycol et 6% poids d'oligomères est soutiré du quatrième réacteur et partiellement condensé puis envoyé à la section de purification du MEG.

L'effluent liquide du quatrième réacteur est envoyé dans un dernier réacteur (réacteur de polycondensation) opéré à 280°C et 0,000013 MPa avec un temps de séjour de 1 h. Un effluent vapeur de composition 57% poids de MEG et 43% poids d'eau est soutiré et partiellement condensé puis envoyé à la section de purification du MEG.

La section de purification du MEG comprend une première colonne de distillation munie de 25 plateaux opérée en tête à 145°C et 0,02 MPa permettant de séparer le diéthylène glycol. Le fond de la première colonne de distillation est envoyé dans une seconde colonne de distillation munie de 17 plateaux opérée en tête à 100°C et 0,1 MPa permettant de séparer les composés légers tel que l'eau et l'acétaldéhyde. Le MEG récupéré à l'issu de ces 2 distillations présente une pureté supérieure à 99,8% et est ensuite recyclé vers le ballon mélangeur. 6,25 t/h de PET sont produits. La consommation globale en énergie primaire de la production de PET est de 5,8 MMkcal/h.

### EXEMPLE 2 - Selon l'invention

### Production de BHET par glycolyse de paillette PET à recycler

4 t/h de paillettes issues d'une charge PET à recycler, broyée et lavée, constituée à 50% poids de PET opaque et 50% poids de PET coloré, sont fondus dans une extrudeuse à 250°C et mélangés avec 11,4 t/h d'éthylène glycol (MEG). Le mélange obtenu est injecté dans un réacteur agité, maintenu à 220°C et à une pression de 0,4 MPa, pendant un temps de séjour de 4h. A la sortie du réacteur, l'effluent réactionnel comprend 66% poids de MEG, 27,4% poids de BHET, 1,7% poids de diéthylène glycol (DEG), 0,2% poids d'eau et 4,7% poids d'oligomères, pigments et autres composés lourds.

L'éthylène glycol présent dans l'effluent réactionnel est séparé par évaporation dans une succession de 5 ballons à des températures allant de 200°C à 124°C et des pressions de 0,1 MPa à 0,00025 MPa. A l'issue de cette étape d'évaporation, un flux de MEG de 10,95 t/h composé de 97% poids de MEG, 2,5% poids de DEG, 0,2% poids d'eau et 0,2% poids BHET, et un flux liquide riche en BHET de 5,17 t/h sont récupérés. Le flux de MEG est envoyé dans une première distillation munie de 25 plateaux et opérée en tête à 0,02 MPa et 145°C pour séparer le DEG et produits lourds puis dans une seconde colonne de distillation munie de 17 plateaux et opérée en tête à 100°C et 0,1 MPa pour séparer l'eau et récupérer un effluent MEG purifié qui peut ensuite être recyclé vers le réacteur de dépolymérisation en mélange avec un appoint de MEG frais. Le flux liquide riche en BHET comprend 87,1% poids de BHET, 0,2% poids de MEG, 0,1% poids de DEG et 12,6% poids d'oligomères, pigments et autres composés lourds.

Le flux liquide riche en BHET est ensuite injecté dans une distillation court trajet à une température de 205°C et une pression de 0,00002 MPa. Un effluent liquide de BHET pré-purifié avec un débit de 4,46 t/h est récupéré en refroidissant les vapeurs dans la distillation court-trajet à 115°C. Il comprend 99,8% poids de BHET, 0,1 % poids de MEG et 0,1 % poids de DEG. Un résidu lourd comprenant 93% poids d'oligomères, pigments et autres composés lourds et 7% poids de BHET est également récupéré à un débit de 0,7 t/h à la sortie de la distillation court trajet.

Le flux liquide de BHET pré-purifié est comprimé jusqu'à 0,5 MPa puis alimente un lit fixe de charbon actif ayant une capacité d'adsorption égale à 5% de sa masse. A l'issue de cette étape, un flux liquide de BHET décoloré et dépigmenté est récupéré et réinjecté dans une étape de préparation d'un mélange des monomères comme celle décrite dans l'exemple 1. Le mélange préparé subit ensuite les différentes étapes de polymérisation comme dans le procédé décrit dans l'exemple 1 en vue de produire du PET.

Le tableau 1 ci-dessous reporte les quantités des monomères PTA, MEG et de monomère BHET solide incorporé, les taux de solide dans le mélange des charges obtenu à 110°C, le rapport du nombre de motif diol sur le nombre de motif téréphtalate pour la production de 6,25 t/h de PET tenant compte de l'incorporation de BHET issu du procédé de dépolymérisation décrit ci-dessus, pour deux rapports de motifs de diol sur les motifs de téréphtalate (1,23 et 1,1). Les résultats présentés sont des résultats calculés, pour différentes quantités de BHET introduites dans le mélange, en considérant que 1 mol de BHET remplace dans le mélange 1 mol de PTA et 2 mol de MEG et basés sur des simulations procédés intégrant des données de solubilité et des données thermodynamiques calées sur des points expérimentaux.

| | | Exemple 1 | Exemple 2a | Exemple 2b | Exemple 2c |
|---|---|---|---|---|---|
| Quantité de PET produit | [t/h] | 6,25 | 6,25 | 6,25 | 6,25 |
| Quantité de PTA | [t/h] | 5,5 | 4,36 | 2,76 | 4,36 |
| Quantité de MEG (frais + recycle) | [t/h] | 2,5 | 1,65 | 0,48 | 1,39 |
| Quantité de BHET incorporé | [t/h] | 0 | 1,74 | 4,2 | 1,74 |
| Rapport motifs diol/motifs téréphtalate | [mol/mol] | 1,23 | 1,23 | 1,23 | 1,1 |
| Taux de solide | [% vol] | 60,7 | 49,2 | 31,8 | 51,5 |

Il apparait que le taux de solide est sensiblement réduit lorsque du BHET est introduit dans les mélanges diphasiques de monomères destinés à la polymérisation (charges d'estérification) des exemples 2a, 2b, 2c par rapport au taux de solide d'un mélange diphasiques de monomères de l'exemple 1 : réduction du taux volumique de solide d'environ 15% à env. 48%. Plus particulièrement, à rapport de motifs diol sur motif téréphtalate équivalent, le taux de solide entre le mélange de l'exemple 1 et des exemples 2a et 2b passe de 60,7% vol. à respectivement 49,2% volumique (baisse d'environ 19%) et 31,8% volumique (baisse d'environ 48%), en fonction de la quantité de BHET introduite dans le mélange de monomères. L'exemple 2c, en comparaison avec l'exemple 2a, montre que pour une même quantité de BHET introduite dans le mélange de monomères (1,74 t/h), il est possible de préparer un mélange diphasique de monomère avec taux de solide réduit par rapport à un mélange ne comprenant pas de BHET (51,5% volumique par rapport à 60,7% volumique dans l'exemple 1) tout en diminuant la quantité d'éthylène glycol apporté (seulement 1,39 t/h dans l'exemple 2c par rapport à 1,65 t/h dans l'exemple 2a). Ainsi, la consommation en matière première éthylène glycol est diminuée sans que cela soit préjudiciable à la qualité du mélange diphasique, lequel peut être facilement transporté vers les opérations de polymérisation.

## Revendications

1. Procédé de production d'un polyester téréphtalate, comprenant :
a) une étape de préparation d'une charge d'estérification comprenant au moins une section de mélange alimentée en au moins une charge acide téréphtalique et une charge monomère diester, les quantités en au moins ladite charge acide téréphtalique et ladite charge monomère diester, introduites dans ladite section de mélange dans ledit mélange étant ajustées de sorte que le rapport du nombre total de moles de motifs diol de formule - [C₍ₙ₊₁₎H₍₂ₙ₊₂₎O₂]-, n étant un nombre entier supérieur ou égal à 1, introduits dans ladite section de mélange, par rapport au nombre total de moles de motifs téréphtalate de formule -[CO-(C₆H₄)-CO]-, introduits dans ladite section de mélange, est compris entre 1,0 et 2,0, ladite section de mélange étant opérée à une température comprise entre 25 et 250°C et à une pression supérieure ou égale à 0,1 MPa,
b) une étape d'estérification de ladite charge d'estérification issue de l'étape a), pour produire au moins un effluent réactionnel et un effluent aqueux, ladite étape d'estérification comprenant au moins une section réactionnelle opérée à une température entre 150 et 400°C, à une pression entre 0,05 et 1 MPa, et avec un temps de séjour entre 1 et 10 h, et au moins une section de séparation,
c) une étape de polycondensation dudit effluent réactionnel obtenu à l'étape b) pour obtenir au moins ledit polyester téréphtalate et un effluent diol, ladite étape comprenant au moins une section réactionnelle comprenant au moins un réacteur dans lequel est mise en oeuvre la polycondensation et étant opérée à une température entre 200 et 400°C, à une pression entre 0,0001 et 0,1 MPa, avec un temps de séjour entre 0,1 et 5 h, ladite section réactionnelle comprenant également au moins un soutirage d'un effluent diol,
d) une étape de traitement des diols, comprenant une section de récupération alimentée au moins par tout ou partie de l'effluent diol issu de l'étape c), pour obtenir un effluent diol à traiter et une section de purification dudit effluent diol à traiter pour obtenir un flux diol purifié.

2. Procédé de production selon la revendication 1, dans lequel le rapport du nombre total de moles de motifs diol de formule -[C₍ₙ₊₁₎H₍₂ₙ₊₂₎O₂]-, introduits dans ladite section de mélange de l'étape a), par rapport au nombre total de moles de motifs téréphtalate de formule -[CO-(C₆H₄)-CO]-, introduits dans ladite section de mélange de l'étape a), est compris entre 1,0 et 1,5, de manière préférée entre 1,0 et 1,3.

3. Procédé de production selon l'une des revendications précédentes, dans lequel ladite section de mélange de ladite étape a) est en outre alimentée en une charge monomère diol qui comprend de préférence au moins 70% mol., préférentiellement au moins 90% mol., de manière très préférée 99,5% mol., d'un monomère diol entant dans la composition du motif unitaire dudit polyester téréphtalate.

4. Procédé de production selon l'une des revendications précédentes, dans lequel ladite charge monomère diester de l'étape a) comprend au moins une fraction d'un effluent diester purifié obtenu à l'issue d'un procédé de dépolymérisation d'une charge polyester à recycler.

5. Procédé de production selon la revendication précédente, dans lequel ledit effluent diester purifié est obtenu par un procédé de dépolymérisation d'une charge polyester à recycler comprenant au moins les étapes suivantes :
i) une étape de dépolymérisation comprenant au moins une section réactionnelle alimentée en ladite charge polyester à recycler et en un flux glycol, pour obtenir un effluent réactionnel de dépolymérisation,
ii) une étape de séparation-purification, comprenant une section de séparation pour obtenir un effluent glycol et une section de purification pour obtenir un effluent diester purifié,
iii) une étape de recyclage d'au moins une fraction dudit effluent diester purifié obtenu à l'étape ii) vers ladite étape a).

6. Procédé de production selon l'une des revendications précédentes, dans lequel ladite charge diester dans la section de mélange de l'étape a) est sous forme liquide.

7. Procédé de production selon la revendication précédente, dans lequel ladite étape de préparation comprend une section de conditionnement, située en amont de la section de mélange, alimentée au moins en une charge diester sous forme solide et opérée à une température comprise entre 25 et 250°C et à une pression supérieure ou égale à 0,1 MPa.

8. Procédé de production selon l'une des revendications précédente, dans lequel ledit polyester téréphtalate est le polyéthylène téréphtalate et ledit monomère diester est le bis(2-hydroxyethyl) téréphtalate (BHET).
